# EUROPEAN PATENT APPLICATION

(11) **EP 4 516 335 A1**
(43) Date of publication of application: **05.03.2025**
(21) Application number: 23306428.6
(22) Date of filing: 29.08.2023
(51) Int. Cl.: A61M 5/00

(54) **PACKAGING SYSTEM FOR TRANSPORTING MEDICAL DEVICES AND METHOD OF FORMING SUCH A PACKAGING SYSTEM**

(71) Applicant: Becton Dickinson France, 38800 Le Pont-de-Claix (FR)
(72) Inventor: REMPFER, Simon, 38260 SAINT-HILAIRE-DE-LA-CÔTE (FR); MAGGUILI, Thomas, 38220 VIZILLE (FR); LAVIGNE, Ferdinand, 38170 SEYSSINET PARISET (FR); LE LOC'H, Clémentine, 38240 MEYLAN (FR); PINEDA, Florian, 38000 GRENOBLE (FR); SOUSA, Hugo, 38170 SEYSSINET PARISET (FR)
(74) Representative: Regimbeau

(57) **Abstract**

A packaging system for receiving medical containers or components of medical containers comprises a plate, a tub having a plurality of sidewalls defining an interior space in which the plate is received, and a material located between sidewalls of the plate and interior surfaces. The material is distinct from the plate and the tub and formed of a material having a composition different from each of a composition of the plate and a composition of the tub.

## Description

### TECHNICAL FIELD

The present disclosure relates generally to a packaging system comprising a device for transporting medical containers, such as syringes, and/or components thereof, such as stoppers, and an associated container in which the device is received.

### BACKGROUND

Medical containers, such as pre-fillable or prefilled syringes, vials, and ampoules, and components for use with these containers, such as stoppers, may be transported from one site to another site. For instance, medical containers may be transported from a manufacturing site to a site at which the medical containers may be filled with a medicinal fluid. By way of further example, the medical containers may be manufactured and filled at the same site and subsequently transported to a storage site in a sterile packaging. During transportation, the medical containers may be supported by a holding device, which may be referred to as a nest. One or more holding devices may be disposed within a packaging having an opening allowing for placement of the holding devices therein and subsequently sealed by a sealing cover. The container may be referred to as a tub.

External dimensions of the holding devices being slightly smaller than inner dimension of the packaging for ease of the assembly, the holding device may be susceptible to moving relative to the packaging during transportation. As a result of such movement during long-distance transport, the holding device rubs against an inner surface of the packaging and may wear away material of the packaging or of the holding device so as to create particles of the packaging material and/or the holding device material. Because the packaging is sealed by the sealing cover, the particles remain inside the packaging throughout the remainder of transportation and storage. Such particles may be dispersed within the packaging and thus disposed within or on the medical containers stored inside the packaging. When the medical containers arrive at the destination, the medical containers are intended to be in a ready-to-use condition. By way of non-limiting example, when the medical containers are pre-fillable syringes, the syringes are intended to be in a clean and sterile condition upon arrival at the filling site so that the syringes may be filled with medicinal fluid. If the particles of the packaging material and/or of the holding device material are dispersed within or on the syringes, the syringes are not in a ready-to-use condition and must be cleaned prior to filling in order to avoid contamination of the medicinal fluid with which the medical containers will be filled. Accordingly, there is a need for a packaging solution that would inhibit or altogether prevent the creation of particles within the packaging.

### BRIEF SUMMARY

An objective of the present disclosure is to provide a packaging system for receiving a plurality of medical containers or components of medical containers solving the above-mentioned drawbacks. The packaging system comprises:
- a plate having an upper surface, a lower surface, and a sidewall extending between the upper surface and the lower surface, the plate defining a plurality of openings extending therethrough from the upper surface to the lower surface, each opening configured to retain a medical container or component of a medical container therein, the plate comprising a plurality of projections that each define a portion of the sidewall;
- a tub having a plurality of sidewalls defining an interior space configured to receive the plate therein, the tub comprising a plurality of projections that each define a portion of an interior surface of the tub; and
- a material located between the portion of the sidewall of the plate defined by the projections and the portion of the interior surface of the tub defined by the plurality of projections, the material being distinct from the plate and the tub and formed of a material having a composition different from each of a composition of the plate and a composition of the tub.

Certain preferred but non-limiting features of the packaging system described above are the following, taken individually or in combination:
the material is coupled to the portion of the interior surface of the tub defined by the plurality of projections or to the portion of the sidewall of the plate defined by the projections;
the composition of the material has hardness less than a hardness of each of the composition of the tub and the composition of the plate;
the material comprises a thermoplastic elastomer, rubber or liquid silicone rubber;
the material comprises a coating applied to the portion of the interior surface of the tub defined by the plurality of projections or to the portion of the sidewall of the plate defined by the plurality of projections;
the material of the coating comprises one of polytetrafluoroethylene, polyether ether ketone, polyvinylidene fluoride, polyurethane, and polyoxymethylene;
the material is a lubricant material, preferably silicone oil, applied to the portion of the interior surface of the tub defined by the plurality of projections and/or to the portion of the sidewall of the plate defined by the projections;
the plurality of projections define a minority of a periphery of the plate, a remainder of the periphery of the plate is recessed relative to the portion of the sidewall defined by the projections such that, when the plate is disposed in the tub, a space is provided between the remainder of the sidewall and the interior surface of the tub, and the remainder of the sidewall is free of the material;
the material is a sheet made of synthetic fibers and the material extends about a periphery of the plate.

Another objective of the present disclosure is to provide a method of forming a packaging system for receiving a plurality of medical containers or components of medical containers, comprising disposing a material on one of:
- a portion of a sidewall of a plate that extends between an upper surface and a lower surface thereof and that is defined by a plurality of projections, and
- a portion of a sidewall of a tub that defines an interior space configured to receive the plate therein and that comprises a plurality of projections that each define a portion of an interior surface of the tub,
wherein the material is formed from a material having a composition different from each of a composition of the plate and a composition of the tub.

Certain preferred but non-limiting features of the method described above are the following, taken individually or in combination:
disposing the material comprises sequentially molding or bonding the material on one of the portion of the interior surface of the tub defined by the plurality of projections or to the portion of the sidewall of the plate defined by the plurality of projections;
disposing the material comprises forming the material by dipping, painting, or spraying the material on one of the portion of the interior surface of the tub defined by the plurality of projections or to the portion of the sidewall (106) of the plate (100) defined by the plurality of projections (108).
the material comprises a sheet formed of synthetic fibers, and disposing the material comprises disposing the sheet within the interior space of the tub prior to disposing the plate within the interior space of the tub;
disposing the material comprises applying a layer of silicone oil to the portion of the interior surface of the tub defined by the plurality of projections and/or to the portion of the sidewall of the plate defined by the plurality of projections.

### BRIEF DESCRIPTION OF THE DRAWINGS

The illustrations presented herein are not meant to be actual views of any particular component, device, or system, but are merely idealized representations which are employed to describe embodiments of the present invention. Other features, goals, and advantages of the present invention will appear more clearly upon reading of the detailed description that follows and with references to drawings provided by way of non-limiting examples wherein:
FIGS. 1 and 2 are views of a plate for a packaging system;
FIGS. 3 and 4 are perspective views of a tub for a packaging system; and
FIG. 5 is a top view of a packaging system including the tub and plate.

### DETAILED DESCRIPTION

As used herein, relational terms, such as "first," "second," "top," "bottom," "upper," "lower," "over," "under," "on," "interior," "exterior," and the like, are used for clarity and convenience in understanding the disclosure and accompanying drawings and do not connote or depend on any specific preference, orientation, or order, except where the context clearly indicates otherwise.

As used herein, the terms "axial," "axially," "longitudinal," and "longitudinally" generally mean and refer to a direction along or parallel to a longitudinal axis of an element(s) of the plate and packaging described herein.

As used herein, the terms "lateral" and "laterally" generally mean and refer to a direction perpendicular to the central, longitudinal axis of the element(s) of the plate and packaging described herein.

As used herein, the term "and/or" includes any and all combinations of one or more of the associated listed items.

As used herein, the term "configured" refers to a size, shape, material composition, material distribution, orientation, and arrangement of one or more of at least one structure and at least one apparatus facilitating operation of one or more of the structure and the apparatus in a pre-determined way.

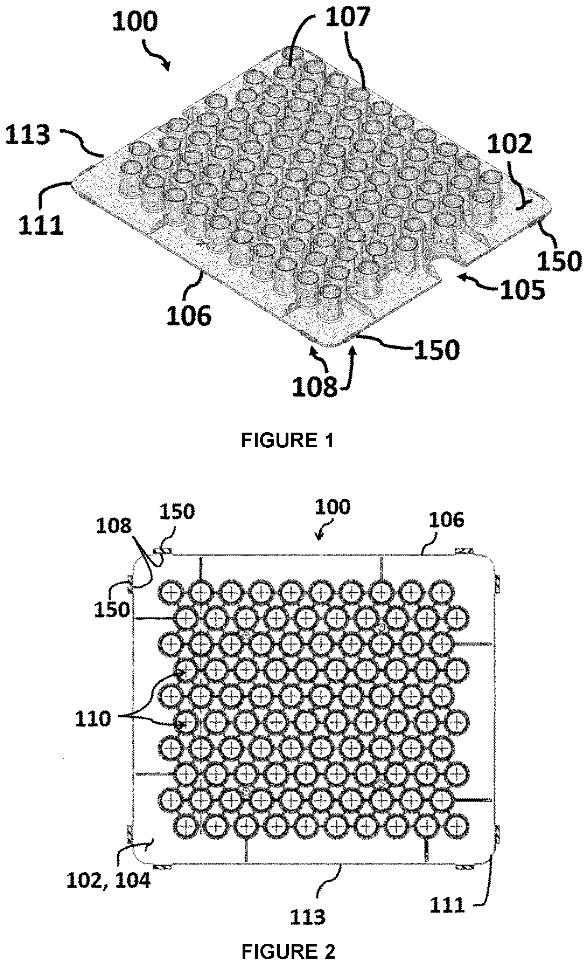

FIGS. 1 and 2 illustrate devices configured to support a plurality of medical containers or a plurality of components of a medical container therein. The device may be referred to in the art generally as a nest. The medical containers may be vials, syringes, such as pre-fillable or prefilled syringes, and the like. The medical containers are configured, after filling, to contain a medical fluid therein. The components of the medical container may be stoppers, syringe gaskets, and the like.

The device comprises a plate 100 having an upper surface 102, a lower surface 104, and a sidewall 106 extending between the upper surface 102 and the lower surface 104.

The sidewall 106 defines a perimeter of the plate 100. Generally, the sidewall 106 comprises four edges 113 connected by corners 111, which may be square or rounded. In some embodiments, indentations 105 may be formed in edges 113 of the plate 100. Such indentations 105 are configured to be gripped by a person or a machine to facilitate insertion and removal the plate 100 from a tub 130.

The plate 100 may comprise a plurality of projections 108 that respectively define some portion of the sidewall 106. The projections 108 are those portions of the plate 100 having the greatest lateral dimensions (e.g., a width or length). The plurality of projections 108 define a minority of the periphery of the plate 100, and a remainder of the periphery of the plate 100 is recessed relative to the portion of the sidewall 106 defined by the projections 108. When the plate 100 is disposed in the tub 130, a space is provided between the remainder of the sidewall 106 and the interior surface 136 of the tub 130. In other embodiments, the plate 100 may lack projections such that each edge of the plate 100 is substantially planar.

A longitudinal axis of the plate 100 extends perpendicular to (e.g., normal to) the upper surface 102. Apart from features such as chimneys 107, the upper surface 102 of the plate 100 is substantially planar. The lower surface 104 may also be substantially planar.

With continued reference to FIGS. 1 and 2, the plate 100 defines a plurality of openings 110 extending therethrough from the upper surface 102 to the lower surface 104. Each opening 110 is configured to retain a medical container or component of a medical container therein.

The plate 100 may also comprise a plurality of chimneys 105 that protrude axially from the upper surface 102. Each chimney 105 extends at least partially circumferentially about a corresponding opening 110. Each chimney 105 may be a generally cylindrical structure. Each chimney 105 defines an opening within which a medical container may be retained. Each chimney 105 may have a centrally extending longitudinal axis that is parallel with the longitudinal axis of the plate 100. Each chimney 105 may be integrally formed with the plate 100.

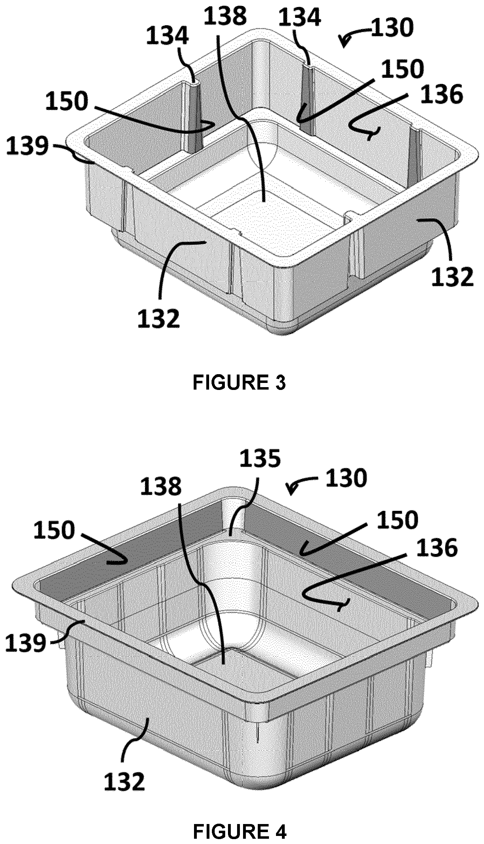

FIGS. 3 and 4 illustrate exemplary embodiments of tubs 130 in which one or more plates 100 may be received. The tub 130 having one or more plates 100 disposed therein collectively form a packaging system 200 for receiving and transporting a plurality of medical containers or components of medical containers.

With continued reference to FIGS. 3 and 4, the tub 130 comprises a plurality of sidewalls 132. The sidewalls 132 may extend from a bottom wall 138 that defines a base of the tub 130. The interior of the tub 130 is hollow and is defined by an interior surface of the bottom wall 138 and an interior surface 136 of the sidewalls 132.

Opposite the bottom wall 138, the tub 130 is open such that the plates 100 may be inserted therein. A flange 139 may be provided at an end of the sidewalls 132 opposite to which the bottom wall 138 is located. After one or more plates 100 having medical containers or medical container components therein are disposed in the tub 130, a sealing element may be coupled to a surface of the flange 139 to close the opening of the tub 130 and allow for sterilization of the packaging system 200.

As illustrated in FIG. 3, the tub 130 may comprise a plurality of projections 134 that respectively define a portion of the interior surface 136 of the tub 130. The plurality of projections 134 define a minimum lateral dimension (e.g., width or length) measured between opposing sidewalls 132. A remainder of the interior surface 136 defined by the sidewalls 132 is recessed relative to the interior surface 136 defined by the projections 134.

The projections 108 of the plate 100 and the projections 134 of the tub 130 are respectively located such that when the plate 100 is disposed in the tub 130, the projections 108, 134 are collocated. Put differently, the plate 100 and the tub 130 are designed such that, absent some material therebetween, the projections 108 of the plate 100 and the projections 134 of the tub 130 are in direct contact while a remainder of the sidewall 106 of the plate 100 and a remainder of the interior surface 136 of the tub 130 are separated from each other. This contact between the tub 130 and the plate 100 and rubbing therebetween may result in the production of particles as previously described herein.

In other embodiments, the tub 130 lacks projections 134. As illustrated in FIG. 4, the sidewalls 132 may be stepped to define a ledge 135. The ledge 135 provides a horizontal surface configured to support the plate 100. When the plate 100 is disposed in the tub 130, the lower surface 104 of the plate 100 is disposed on the ledge 135. The ledge 135 may extend continuously or discontinuously along a perimeter of the interior surface 136. When the plate 100 is disposed in the tub 130, absent some material therebetween, at least a portion of the sidewall 106 of the plate 100 contacts the interior surface 136 of the tub 130. This contact between the tub 130 and the plate 100 and rubbing therebetween may result in the production of particles as previously described herein. There is also the possibility of having a contact between the projections 108 of the plate and the sidewalls of the tub, even if said sidewalls do not have projections.

A material 150 having a composition different from a composition of each of the tub 130 and the plate 100 is disposed on the tub 130 and/or the plate 100 as described in further detail below. The tub 130 and/or the plate 100 may be formed of a plastic, such as a polyolefin including polyethylene or polypropylene, polystyrene, polycarbonate, polyester, or polyamide.

The material 150 is located at an interface between the tub 130 and the plate 100 so as to prevent direct contact therebetween.

FIGS. 1 and 2 illustrate embodiments in which the material 150 is disposed on the plate 100. In particular, the material 150 may be disposed on each of the projections 108 of the plate 100. In some embodiments, the material 150 is disposed only on the projections 108 such that a remainder of the sidewall 106 is free of material. In other embodiments, the material 150 may be provided along the sidewall 106 such that the entire perimeter of the plate 100 includes the material 150.

FIGS. 3 and 4 illustrate embodiments in which the material 150 is disposed on the tub 130. In particular, the material 150 may be disposed on a portion of the interior surface 136 of the sidewalls 132 of the tub 130.

In the embodiment illustrated in FIG. 3, the material 150 is disposed on the interior surface 136 of the tub 130 defined by the projections 134. The material 150 may be provided along a full height of the projection 134 between the flange 139 and the bottom wall 138. A remainder of the interior surface 136 of the tub 130 is free of the material 150.

In the embodiment illustrated in FIG. 4, the material 150 is disposed on the interior surface 136 of the tub 130 defined by the portion of the sidewalls 132 located extending axially above the ledge 135. The material 150 may extend continuously or discontinuously along the respective sidewalls 132. For example, the material 150 may be disposed on those surfaces of the sidewall 132 extending parallel to the edges 113 of the plate 100 when the plate 100 is disposed in the tub 130. Although not illustrated in FIG. 4, the material may also extend on the ledge 135.

With reference to FIG. 3, the material 150 is disposed on each of the projections 134 of the tub 130. In some embodiments, the material 150 is disposed only to the projections such that a remainder of the interior surface of the tub 130 is free of material.

In some embodiments, the material 150 may be coupled to the plate 100 or the tub 130 by an adhesive or by bonding. Bonding may be accomplished by melt adhesion during a sequential injection molding process, also known as bi-injection process (e.g. overmolding or comolding). Alternatively, the material 150 may be provided separately and assembled to the tub 130 or to the plate 100.

The material 150 coupled to the plate 100 or tub 130 by adhesive or bonding is selected to have a composition having a hardness less than a hardness of the composition of the tub 130 and the composition of the plate 100. The material 150 may have hardness on the Shore A Hardness Scale in a range from 20 to 75. The material 150 may comprise a thermoplastic elastomer, rubber or liquid silicone rubber. The Shore D Hardness of the plate 100 and/or tub 130 may be between 50 and 100. By virtue of the presence of the relatively softer material 150 between the plate 100 and the tub 130, particles of the material of the plate 100, the tub 130, and the material 150 are not created when the plate 100 having the material 150 thereon rubs against the interior surface 136 of the tub 130 or when the plate 100 rubs against the interior surface 136 of the tub 130 having the material 150 thereon during transportation.

In further embodiments of the disclosure, the material 150 may comprise an abrasion-resistant coating applied to portions of the interior surface 136 of the tub 130 or portions of the sidewall 106 of the plate 100.

The abrasion-resistant coating may be a plastic coating. The material 150 may comprise one of polytetrafluoroethylene, polyether ether ketone, polyvinylidene fluoride, polyurethane, and polyoxymethylene. The abrasion resistance of the material 150 is sufficient such that the material is not abraded by the material of the tub 130 or plate 100. By virtue of the presence of the abrasion-resistant material 150 located between the plate 100 and the tub 130, particles of the material of the plate 100, the tub 130, and the material 150 are not created when the plate 100 having the material 150 thereon rubs against the interior surface 136 of the tub or when the plate 100 rubs against the interior surface 136 of the tub 130 during transportation.

The material 150 may be applied to the tub 130 or plate 100 by painting, spraying, or dipping. Said application may be done locally (e.g. only applied to the portions of the tub and plate that contact each other), in particular by using a mask to cover the portions of the tub or plate on which it is not intended to apply the material.

In the previously described embodiments, the material 150 is in a solid state. In other embodiments of the disclosure, the material 150 may be provided in a liquid state.

The lubricant material may be silicone oil. By virtue of the presence of the lubricant material located between the plate 100 and the tub 130, particles of the material of the plate 100 and the tub 130 are not created when the plate 100 having the material 150 thereon rubs against the interior surface 136 of the tub or when the tub 130 having the material thereon rubs against the plate 100 during transportation.

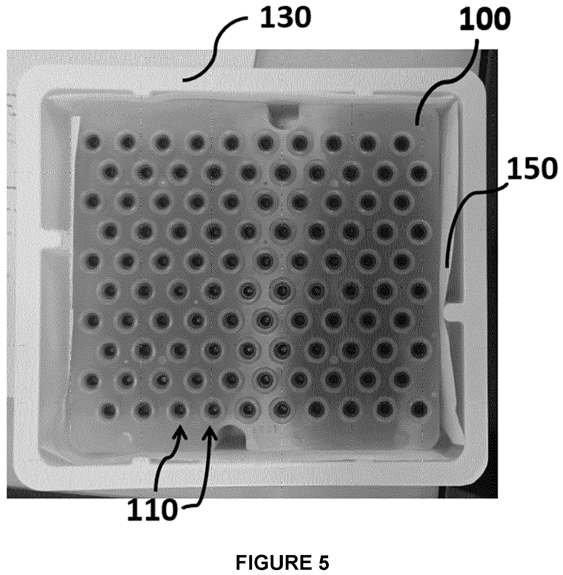

In another embodiment, such as the embodiment illustrated in FIG. 5, the material 150 may be disposed between the plate 100 or tub 130 without being coupled to either the plate 100 or tub 130. In such embodiments, the material 150 may be a sheet of material that is resistant to abrasion by the material of the plate 100 and tub 130. In particular, the material may be a sheet made of synthetic fibers. For example, the sheet may be formed of a spunbonded, synthetic fiber, such as a sheet of TYVEK^{™}.

The sheet is disposed within the interior space of the tub 130 prior to disposing the plate 100 within the tub 130. By virtue of the presence of the sheet located between the plate 100 and the tub 130, particles of the material of the plate 100 and the tub 130 are not created because contact between the tub 130 and the plate 100 is prevented during transportation.

While the present disclosure has been described herein with respect to certain illustrated embodiments, those of ordinary skill in the art will recognize and appreciate that it is not so limited. Rather, many additions, deletions, and modifications to the illustrated embodiments may be made without departing from the scope of the disclosure, including legal equivalents thereof. In addition, features from one embodiment may be combined with features of another embodiment while still being encompassed within the scope of the invention as contemplated by the inventors.

## Claims

1. A packaging system (200) for receiving medical containers or components of medical containers, comprising:
a plate (100) having an upper surface (102), a lower surface (104), and a sidewall (106) extending between the upper surface (102) and the lower surface (104), the plate (100) defining a plurality of openings (110) extending therethrough from the upper surface (102) to the lower surface (104), each opening (110) configured to retain a medical container or component of a medical container therein, the plate (100) comprising a plurality of projections (108) that each define a portion of the sidewall (106);
a tub (130) having a plurality of sidewalls (132) defining an interior space configured to receive the plate (100) therein, the tub (130) comprising a plurality of projections (134) that each define a portion of an interior surface (136) of the tub (130); and
a material (150) located between the portion of the sidewall (106) of the plate (100) defined by the projections (108) and the portion of the interior surface (136) of the tub (130) defined by the plurality of projections (134), the material (150) being distinct from the plate (100) and the tub (130) and formed of a material having a composition different from each of a composition of the plate (100) and a composition of the tub (130).

2. The packaging system of claim 1, wherein the material (150) is coupled to the portion of the sidewall (106) of the plate (100) defined by the projections (108) or to the portion of the interior surface (136) of the tub (130) defined by the plurality of projections (134).

3. The packaging system of either claim 1 or 2, wherein the composition of the material (150) has hardness less than a hardness of each of the composition of the tub (130) and the composition of the plate (100).

4. The packaging system of any of claims 1-3, wherein the material (150) comprises a thermoplastic elastomer, rubber or liquid silicone rubber.

5. The packaging system of claim 1, wherein the material (150) comprises a coating applied to the portion of the interior surface (136) of the tub (130) defined by the plurality of projections (134) or to the portion of the sidewall (106) of the plate (100) defined by the plurality of projections (108).

6. The packaging system of claim 4, wherein the material (150) of the coating comprises one of polytetrafluoroethylene, polyether ether ketone, polyvinylidene fluoride, polyurethane, and polyoxymethylene.

7. The packaging system of claim 1, wherein the material (150) is a lubricant material, preferably silicone oil, applied to the portion of the interior surface (136) of the tub (130) defined by the plurality of projections (134) and/or to the portion of the sidewall (106) of the plate (100) defined by the projections (108).

8. The packaging system of any of claims 1-7, wherein:
the plurality of projections (108) define a minority of a periphery of the plate (100),
a remainder of the periphery of the plate (100) is recessed relative to the portion of the sidewall (106) defined by the projections (108) such that, when the plate (100) is disposed in the tub (130), a space is provided between the remainder of the sidewall (106) and the interior surface (136) of the tub (130), and
the remainder of the sidewall (106) is free of the material (150).

9. The packaging system of claim 1, wherein the material (150) is a sheet made of synthetic fibers and the material (150) extends about a periphery of the plate (100).

10. A method of forming a packaging system for receiving a plurality of medical containers or components of medical containers, comprising
disposing a material (150) on one of:
a portion of a sidewall (106) of a plate (100) that extends between an upper surface (102) and a lower surface (104) thereof and that is defined by a plurality of projections (108), and
a portion of a sidewall (132) of a tub (130) that defines an interior space configured to receive the plate (100) therein and that comprises a plurality of projections (134) that each define a portion of an interior surface (136) of the tub (130),
wherein the material (150) is formed from a material having a composition different from each of a composition of the plate (100) and a composition of the tub (130).

11. The method of claim 10, wherein disposing the material (150) comprises overmolding, co-molding, or bonding the material (150) on one of the portion of the interior surface (136) of the tub (130) defined by the plurality of projections (134) or to the portion of the sidewall (106) of the plate (100) defined by the plurality of projections (108).

12. The method of claim 10, wherein disposing the material (150) comprises forming the material (150) by dipping, painting, or spraying the material (150) on one of the portion of the interior surface (136) of the tub (130) defined by the plurality of projections (134) or to the portion of the sidewall (106) of the plate (100) defined by the plurality of projections (108).

13. The method of claim 10, wherein the material (150) comprises a sheet (160) formed of synthetic fibers, and wherein disposing the material (150) comprises disposing the sheet (160) within the interior space of the tub (130) prior to disposing the plate (100) within the interior space of the tub (130).

14. The method of claim 10, wherein disposing the material (150) comprises applying a layer of silicone oil to the portion of the interior surface (136) of the tub (130) defined by the plurality of projections (134) and/or to the portion of the sidewall (106) of the plate (100) defined by the plurality of projections (108).
